# EUROPÄISCHE PATENTSCHRIFT

(11) **EP 1 558 556 B1**
(45) Veröffentlichungstag und Bekanntmachung des Hinweises auf die Patenterteilung: **18.03.2009**
(21) Anmeldenummer: 03769425.4
(22) Anmeldetag: 21.10.2003
(51) Int. Cl.: C07C 51/54, C07C 51/09, C07C 67/10, C07C 51/56, C07C 53/122, C07C 53/124, C07C 63/313, C07C 53/02, C07C 69/14, C07C 53/12

(54) **FLEXIBLES VERFAHREN ZUR GEMEINSAMEN HERSTELLUNG VON (I) AMEISENSÄURE,(II) EINER CARBONSÄURE MIT MINDESTENS ZWEI KOHLENSTOFFATOMEN UND/ODER DEREN DERIVATEN UND (III) EINES CARBONSÄUREANHYDRIDS**
FLEXIBLE METHOD FOR THE JOINT PRODUCTION OF (I) FORMIC ACID, (II) A CARBOXYLIC ACID COMPRISING AT LEAST TWO CARBON ATOMS AND/OR THE DERIVATIVES THEREOF, AND (III) A CARBOXYLIC ACID ANHYDRIDE
PROCEDE SOUPLE PERMETTANT DE PRODUIRE CONJOINTEMENT (I) DE L'ACIDE FORMIQUE, (II) UN ACIDE CARBOXYLIQUE COMPORTANT AU MOINS DEUX ATOMES DE CARBONE ET/OU SES DERIVES ET (III) UN ANHYDRIDE D'ACIDE CARBOXYLIQUE

(30) Priorität: 26.10.2002 DE 10249928
(43) Veröffentlichungstag der Anmeldung: 03.08.2005
(73) Patentinhaber: BASF SE, 67056 Ludwigshafen (DE)
(72) Erfinder: PASTRE, Jörg, 64625 Bensheim (DE); EXNER, Kai Michael, 69214 Eppelheim (DE); STÜER, Wolfram, 68165 Mannheim (DE); GEISSLER, Elke, 67069 Ludwigshafen (DE); MACHHAMMER, Otto, 68163 Mannheim (DE)
(86) Internationale Anmeldenummer: PCT/EP2003/011622
(87) Internationale Veröffentlichungsnummer: WO 2004/037762

(56) Entgegenhaltungen:
- EP-A- 0 004 641
- EP-A- 0 087 870
- EP-A- 0 193 799
- EP-A- 0 196 520
- EP-A- 0 336 216
- DE-A- 3 644 222

## Beschreibung

Die vorliegende Erfindung betrifft ein Verfahren zur gemeinsamen Herstellung von (i) Ameisensäure, (ii) einer Carbonsäure mit mindestens zwei Kohlenstoffatomen und/oder deren Derivaten, wie beispielsweise einem Carbonsäureester oder einem Carbonsäureanhydrid, und (iii) eines weiteren Carbonsäureanhydrids.

Ameisensäure ist eine wichtige und vielseitig einsetzbare Verbindung. Sie wird beispielsweise zum Ansäuren bei der Herstellung von Futtermitteln, als Konservierungsmittel, als Desinfektionsmittel, als Hilfsstoff in der Textil- und Lederindustrie sowie als Synthesebaustein in der chemischen Industrie verwendet.

Im Folgenden seien die wichtigsten Verfahren zur Herstellung von Ameisensäure genannt (siehe Ullmann's Encyclopedia of Industrial Chemistry, 6th edition, 2000 electronic release, Chapter "FORMIC ACID - Production").

Das technisch bedeutendste Verfahren zur Herstellung von Ameisensäure ist die Hydrolyse von Ameisensäuremethylester (Methylformiat) und anschließender Aufkonzentrierung der erhaltenen wässrigen Ameisensäurelösung. Als bekannte Verfahren seien das Kemira-Leonard- und das BASF-Verfahren genannt. Ein großer Nachteil dieser Verfahren ist die Bildung einer wässrigen Ameisensäurelösung infolge des Hydrolyseschritts, welche eine Reihe weiterer Nachteile nach sich zieht. So ist eine aufwendige Aufkonzentrierung der Ameisensäurelösung durch Extraktivrektifikation unter Einsatz eines Schleppmittels erforderlich. Durch die Gegenwart von Wasser ist die zu handhabende wässrige beziehungsweise aufkonzentrierte Ameisensäurelösung höchst korrosionsaggressiv und erfordert den Einsatz teuerer Konstruktionsmaterialien für den betreffenden Anlagenteil. Die genannten Verfahren zeichnen sich somit nachteilig durch hohe Investitions- und Betriebskosten, durch eine technisch aufwendige und umfangreiche Gestaltung der Produktionsanlage, durch einen hohen Energieverbrauch und durch die Existenz eines nicht unerheblichen Restwassergehalts in der aufkonzentrierten Ameisensäure aus.

Bei der Oxidation von Kohlenwasserstoffen, wie beispielsweise Butane oder Naphtha, bildet sich ein breites Produktspektrum, welches auch Ameisensäure enthält und in aufwendiger Art und Weise abgetrennt und aufkonzentriert werden kann. Auch bei diesem Verfahren ist zu dessen Nachteil eine Extraktivrektifikation der Roh-Ameisensäure unter Einsatz eines Schleppmittels erforderlich. Auf die oben genannten Nachteile infolge des Wassergehalts sei verwiesen.

Nach einem älteren Verfahren wird Ameisensäure auch durch Hydrolyse von Formamid, welches über Ammonolyse von Ameisensäuremethylester mit Ammoniak zugänglich ist, gewonnen. Die Hydrolyse erfolgt mit Schwefelsäure und Wasser. Nachteilig bei diesem Verfahren ist der unerwünschte Anfall von Ammoniumsulfat als Koppelprodukt und die Gegenwart von Wasser, was zu den oben genannten Nachteilen führt.

Carbonsäuren, wie Essigsäure und ihre höhermolekularen Homologe und die entsprechenden Anhydride, sind wichtige und vielseitig einsetzbare Verbindungen. Sie werden beispielsweise zur Herstellung von Estern, Carbonsäureanhydriden, als Zusatzstoffe im Polymerbereich oder als Zwischenprodukte bei der Herstellung von Textilchemikalien, Farbstoffen, Kunststoffen, Agrar- und Pharmachemikalien verwendet. Eine besonders hohe Bedeutung zeigen die niedermolekularen Homologe Essigsäure und Propionsäure.

Im Folgenden seien die wichtigsten Verfahren zur Herstellung von Essigsäure und ihrer höhermolekularen Homologe genannt (siehe Ullmann's Encyclopedia of Industrial Chemistry, 6th edition, 2000 electronic release, Chapter "ACETIC ACID - Produktion" und Chapter "CARBOXYLIC ACIDS, ALIPHATIC - Production").

Das technisch bedeutendste Verfahren zur Herstellung von Essigsäure ist die Carbonylierung von Methanol in Gegenwart geeigneter Carbonylierungskatalysatoren, wie beispielsweise Cobalt-, Iridium- oder Rhodium-Carbonyl-Verbindungen. Als bekannte Verfahren seien das BASF- und das Monsanto-Verfahren genannt. Nachteilig bei diesen Verfahren ist die Gegenwart von Wasser im Reaktionsmedium, welches durch die Wassergas-Shift-Reaktion von Wasser und Kohlenmonoxid zu Kohlendioxid und Wasserstoff die Ausbeute an eingesetztem Kohlenmonoxid erniedrigt. Des Weiteren ist aufgrund des Wassergehalts in der destillativen Aufarbeitung ein hoher Energieaufwand nötig. Die genannten Verfahren zeichnen sich ferner durch hohe Investitions- und Betriebkosten sowie durch eine technisch aufwendige und umfangreiche Gestaltung der Produktionsanlage aus.

Bei der Oxidation von Kohlenwasserstoffen, wie beispielsweise Ethan, Butane oder Naphtha, bildet sich ein breites Produktspektrum, welches Essigsäure und gegebenenfalls höhere Homologe enthält und in aufwendiger Art und Weise abgetrennt und aufkonzentriert werden kann, Auf die oben genannten Nachteile infolge des Wassergehalts sei verwiesen.

Die Synthese von Carbonsäuren durch Oxidation der entsprechenden Aldehyde geht auf teures Olefin als Einsatzstoff zurück. So wird Acetaldehyd technisch durch Ethen-Oxidation nach dem Wacker-Verfahren sowie seine höheren Homologe durch Hydroformylierung von Ethen, Propen, etc. gewonnen. Diese Verfahren beruhen daher auf einer wirtschaftlich unattraktiven Rohstoffbasis.

Carbonsäureester, insbesondere Essigsäuremethylester (Methylacetat), stellen wichtige Lösungsmittel dar. Essigsäuremethylester wird beispielsweise zum Lösen von Nitrocellulose oder Acetylcellulose eingesetzt. Essigsäurevinylester findet breite Anwendung in der Herstellung von polymeren und Copolymeren.

Die Verfahren zur Herstellung von Carbonsäureestern sind sehr vielfältig (siehe Ullmann's Encyclopedia of Industrial Chemistry, 6th edition, 2000 electronic release, Chapter "ESTERS, ORGANIC - Production"). Genannt seien die Veresterung von Carbonsäuren mit Alkoholen, die Umsatzung von Carbonsäurechloriden oder Carbonsäureanhydriden mit Alkoholen, die Umesterung von Carbonsäureestern, die Umsetzung von Ketenen mit Alkoholen, die Carbonylierung von Olefinen mit Kohlenmonoxid und Alkoholen, die Kondensation von Aldehyden, die Alkoholyse von Nitrilen und die oxidative Acylierung von Olefinen.

Essigsäurealkylester werden hauptsächlich durch Veresterung von Essigsäure oder Essigsäureanhydrid mit Alkanolen gewonnen. Essigsäuremethylester entsteht des Weiteren als Nebenprodukt bei der Synthese von Essigsäure (siehe Ullmann's Encyclopedia of Industrial Chemistry, 6th edition, 2000 electronic release, Chapter "ACETIC ACID - Production"). Eine weitere Synthesemöglichkeit für Essigsäuremethylester ist die Carbonylierung von Dimethylether (siehe Ullmann's Encyclopedia of Industrial Chemistry, 6th edition, 2000 electronic release, Chapter "ACETIC ANHYDRIDE AND MIXED FATTY ACID ANHYDRIDES - Acetic Anhydride - Production"). Nachteil des letztgenannten Verfahrens ist der Einsatz von teurem Dimethylether.

Essigsäureanhydrid (Acetanhydrid) ist ein wichtiger Synthesebaustein in der chemischen Industrie und wird beispielsweise zur Herstellung von Acetylcellulosen, Acetylsalicylsäure, Acetanilid, Sulfonamiden oder Vitamin B6 eingesetzt.

Im Folgenden seien die wichtigsten Verfahren zur Herstellung von Essigsäureanhydrid genannt (siehe Ullmann's Encyclopedia of Industrial Chemistry, 6th edition, 2000 electronic release, Chapter "ACETIC ANHYDRIDE AND MIXED FATTY ACID ANHYDRIDES - Acetic Anhydride - Production").

Ein technisch bedeutendes Verfahren zur Herstellung von Essigsäureanhydrid ist die Umsetzung von Essigsäure mit Keten, wobei das Keten in einer Vorstufe durch thermische Wasserabspaltung aus Essigsäure gewonnen wird. Nachteilig bei diesem Verfahren ist der sehr hohe Energieverbrauch durch die thermische Keten-Darstellung und der Umgang mit dem äußerst giftigen Keten.

In einem weiteren technisch bedeutenden Verfahren zur Herstellung von Essigsäureanhydrid wird in einer ersten Stufe durch Carbonylierung und Veresterung Methanol zu Essigsäuremethylester umgesetzt und dieser in einer zweiten Stufe zum Essigsäureanhydrid carbohyliert.

Ein weiteres Verfahren zur Herstellung von Essigsäureanhydrid ist die Flüssigphasen-Oxidation von Acetaldehyd. Nachteilig bei diesem Verfahren ist der Einsatz von teurem Acetaldehyd, welcher technisch durch Ethen-Oxidation nach dem Wacker-Verfahren gewonnen wird. Dieses Verfahren beruht daher auf einer wirtschaftlich unattraktiven Rohstoffbasis.

Als weiteres Verfahren zur Herstellung von Essigsäureanhydrid sei die Carbonylierung von Essigsäuremethylester in Gegenwart eines Übergangsmetall-Katalysators genannt. Essigsäuremethylester wird in der Regel als Nebenprodukt bei der Synthese von Essigsäure sowie durch Veresterung von Essigsäure mit Methanol gewonnen.

EP-A 0 087 870 lehrt ein integriertes Verfahren zur Herstellung von Essigsäureanhydrid und Essigsäure aus Methanol und Kohlenmonoxid. In einer ersten Stufe wird Essigsäure mit Methanol zum Essigsäuremethylester verestert, welcher in einer zweiten Stufe in Gegenwart von Wasser zu einem Gemisch enthaltend Essigsäureanhydrid und Essigsäure carbonyliert wird. Das gewonnene Gemisch wird destillativ aufgearbeitet, wobei die erforderliche Menge an Essigsäure der ersten Stufe zugeführt wird. Die restliche Menge an Essigsäure und an Essigsäureanhydrid wird als Produkt abgeführt. Nachteilig bei diesem Verfahren ist die Bildung stöchiometrischer Mengen von Wasser in der Veresterungsstufe und die damit vorhandene Problematik beim Umgang mit wasserhaltiger Essigsäure und deren Aufarbeitung. Auf die oben genannten Nachteile infolge des Wassergehalts sei verwiesen.

Carbonsäureanhydride sind wichtige Ausgangsstoffe für andere Säurederivate und werden des Weiteren auch als Lösungsmittel und als Dehydratisierungsmittel eingesetzt. Carbonsäureanhydride ungesättigter aliphatischer Carbonsäuren, insbesondere der Acrylsäure und Methacrylsäure, sind zudem wichtige Ausgangsverbindungen für die Herstellung von interessanten, auf anderen Herstellungswegen nur schwer zugänglichen Monomeren. Aromatische Carbonsäureanhydride, wie beispielsweise 1,2,4,5-Benzoltetracarbonsäuredianhydrid (Pyromellitanhydrid) oder 3,3',4,4'-Benzophenontetracarboxyldianhydrid, sind wichtige Ausgangsmaterialien für die Herstellung wärmebeständiger Harze, wie etwa von Polyamid- oder Epoxyharzen.

Zur Herstellung der Carbonsäureanhydride sind verschiedene Verfahren bekannt. Eine Übersicht über die drei prinzipiellen Herstellwege findet sich beispielsweise unter dem Stichwort "Säureanhydride" in CD Römpp Chemie Lexikon, Version 1.0, Stuttgart/New York, Georg Thieme Verlag 1995. Bei dem ersten Herstellweg werden die zugrunde liegenden Carbonsäuren eingesetzt und durch den Einsatz wasserentziehender Mittel, wie beispielsweise P₄O₁₀, oder durch Erhitzen Wasser entzogen, wobei sich das Carbonsäureanhydrid bildet. Nachteilig bei diesem Herstellweg ist der Einsatz von energetisch sehr hochwertigen Edukten (z.B. P₄O₁₀) und die Bildung von unerwünschten Koppelprodukten (z.B. Phosphorsäure beim Einsatz von P₄O₁₀). Nachteilig am thermischen Wasserentzug ist die Gefahr zur Bildung unerwünschter Nebenprodukte durch thermische Zersetzung. Beim zweiten Herstellweg werden Säurechloride, wie beispielsweise Acetylchlorid oder Benzoylchlorid, mit den Alkalisalzen der entsprechenden Carbonssäuren umgesetzt. Ein entsprechendes Verfahren ist beispielsweise auch in WO 95/32940 beschrieben. Nachteilig bei diesem Herstellweg ist der Einsatz eines Säurechlorids, welches ein energetisch hochwertiges Edukt darstellt und die Bildung von Alkalichlorid und dem Alkalisalz des eingesetzten Säurechlorids als unerwünschte Koppelprodukte. Beim dritten Herstellweg werden die zugrunde liegenden Carbonsäuren mit Essigsäureanhydrid oder Keten umanhydridisiert. Entsprechende Ausführungen dieses Herstellweges sind beispielsweise in DE-A 35 10 035, EP-A 0 231 689, DE-A 36 44 222 und EP-A 1 231 201 beschrieben. Nachteilig bei diesem Herstellweg ist der Einsatz von Essigsäureanhydrid oder Keten, welche zunächst einmal nach den weiter oben unter Essigsäureanhydrid und Keten beschriebenen energieintensiven Herstellverfahren zu gewinnen sind.

Es bestand daher die Aufgabe, ein Verfahren zur Herstellung von Carbonsäuren und/oder deren Derivaten zu finden, welches die oben genannten Nachteile nicht mehr besitzt, auf einer gut zugänglichen und wirtschaftlich attraktiven Rohstoffbasis basiert, eine einfache und kostengünstige Gestaltung der Anlage ermöglicht (niedrige Investitionskosten), unerwünschte Nebenprodukte infolge Koppelproduktion vermeidet und sich durch einen niedrigen Energieverbrauch und günstige Betriebskosten auszeichnet. Es bestand des Weiteren die Aufgabe, ein Verfahren zu finden, welches bei Bedarf auch die Herstellung wasserfreier Carbonsäuren zugänglich macht und somit die Handhabung weniger korrosionsaggresiver Medien sowie den Einsatz kostengünstigerer Konstruktionsmaterialen ermöglicht und infolge der geringeren Korrosionsaggresivität auch eine höhere Sicherheit bietet. Ferner bestand die Aufgabe, ein Verfahren zu finden, welches allgemein die Herstellung der verschiedensten Carbonsäureanhydride und insbesondere auch die Herstellung ungesättigter Carbonsäureanhydride, wie beispielsweise Acrylsäureanhydrid oder Methacrylsäureanhydrid, ermöglicht.

Demgemäß wurde ein Verfahren zur gemeinsamen Herstellung von
(i) Ameisensäure (III);
(ii) einer Carbonsäure mit mindestens zwei Kohlenstoffatomen (II) und/oder deren Derivaten; und
(iii) eines Carbonsäureanhydrids (VII),
gefunden, das dadurch gekennzeichnet ist, dass man
(a) einen Ameisensäureester (I) mit einer Carbonsäure mit mindestens zwei Kohlenstoffatomen (II) zu Ameisensäure (III) und dem entsprechenden Carbonsäureester (IV) umestert;
(b) mindestens einen Teil des in Schritt (a) gebildeten Carbonsäureesters (IV) zum entsprechenden Carbonsäureanhydrid (V) carbonyliert; und
(c) mindestens einen Teil des in Schritt (b) gebildeten Carbonsäureanhydrids (V) mit einer Carbonsäure (VI) unter Bildung eines Carbonsäureanhydrids (VII) und der Carbonsäure (II) umanhydridisiert.

In Schritt (a) setzt man einen Ameisensäureester (I) mit einer Carbonsäure mit mindestens zwei Kohlenstoffatomen (II) zu Ameisensäure (III) und dem entsprechenden Carbonsäureester (IV) um.

Der einzusetzende Ameisensäureester besitzt die allgemeine Formel (I)

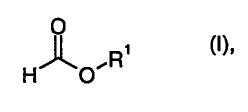

in der der Rest R¹ für einen Kohlenstoff enthaltenden organischen Rest steht. Unter einem Kohlenstoff enthaltenden organischen Rest ist bevorzugt ein unsubstituierter oder substituierter, aliphatischer, aromatischer oder araliphatischer Rest mit 1 bis 12 Kohlenstoffatomen, welcher ein oder mehrere Heteroatome, wie etwa Sauerstoff, Stickstoff oder Schwefel, wie beispielsweise -O-, -S-, -NR-, -CO- und/oder -N= in aliphatischen oder aromatischen Systemen, enthalten kann, und/oder durch eine oder mehrere funktionelle Gruppen, welche beispielsweise Sauerstoff, Stickstoff, Schwefel und/oder Halogen enthalten können, wie beispielsweise durch Fluor, Chlor, Brom, Iod und/oder eine Cyanogruppe, substituiert sein kann, zu verstehen.

Ameisensäureester sind im Allgemeinen zugänglich über eine basenkatalysierte Carbonylierung der entsprechenden Alkohole sowie über eine Veresterung der entsprechenden Alkohole mit Ameisensäure (siehe Ullmann's Encyclopedia of Industrial Chemistry, 6th edition, 2000 electronic release, Chapter "FORMIC ACID - Derivatives"). Der einfachste Vertreter dieser Verbindungsklasse, Ameisensäuremethylester, wird großtechnisch durch Carbonylierung von Methanol gewonnen.

Als Carbonsäure mit mindestens zwei Kohlenstoffatomen (II) ist eine Carbonsäure zu verstehen, welche an der Carboxy-Gruppe einen Rest mit mindestens einem Kohlenstoffatom aufweist. Die einzusetzenden Carbonsäuren besitzen die allgemeine Formel (II)

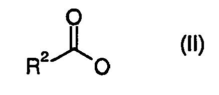

in der der Rest R² für einen Kohlenstoff enthaltenden organischen Rest steht. Der bevorzugte Kohlenstoff enthaltenden organischen Rest R² ist wie bei R¹ definiert.

Bei der genannten Umesterungsreaktion in Schritt (a) handelt es sich um eine Gleichgewichtsreaktion, welche im Allgemeinen durch die Gegenwart eines Katalysators katalysiert wird.

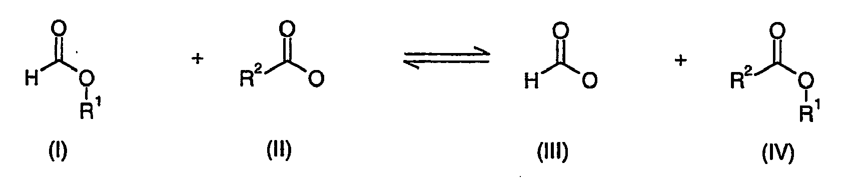

Beim erfindungsgemäßen Verfahren können in Schritt (a) die bekannten Verfahren zur Umesterung eingesetzt werden (siehe Ullmann's Encyclopedia of Industrial Chemistry, 6th edition, 2000 electronic release, Chapter "ESTERS, ORGANIC - Chemical Properties" und "ESTERS, ORGANIC - Production" und die untenstehenden Zitate).

Als Katalysatoren werden im Allgemeinen geringe Mengen saurer oder basischer Substanzen eingesetzt. Bevorzugt ist der Einsatz von Säuren und sauren Festkörpern. Als Beispiele seien starke Protonensäuren, wie beispielsweise Schwefelsäure, Perchlorsäure, Benzolsulfonsäure, p-Toluolsulfonsäure, Molybdatophosphorsäure und Wolframatokieselsäure; saure Ionentauscher, wie beispielsweise perfluorierte Sulfonsäuregruppen enthaltende Ionentauscher (SU-A 1,432,048); sowie saure Oxide, wie beispielsweise Zeolithe (DE-OS 35 06 632), Aluminosilikate (US 3,328,439) oder SiO₂/TiO₂ (DE 2710 630) genannt. Als bevorzugte Katalysatoren seien Mineralsäuren, p-Toluolsulfonsäure und Zeolithe ganannt.

Werden starke Protonensäuren als homogene Katalysatoren eingesetzt, so beträgt deren Konzentration im Reaktionsgemisch im Allgemeinen 0,01 bis 50 Gew.-%, bevorzugt 0,01 bis 2 Gew.-%.

Als Cokatalysator zu den oben genannten Katalysatoren ist der Einsatz von Wasser oder Methanol, in der Regel bis zu 20 Gew.-%, bezogen auf die Reaktionslösung, möglich. Es ist dabei aber zu beachten, dass mit Zunahme des Wassergehalts auch die Korrosionsaggressivität des Reaktionsmediums zunimmt und die Aufarbeitung der Produkte erschwert wird. Daher ist es gegebenenfalls vorteilhaft, die Umesterung ohne Zusatz von Wasser oder Methanol als Cokatalysator durchzuführen. Wird die Umesterung in Gegenwart von Wasser oder Methanol durchgeführt, so ist es gegebenenfalls vorteilhaft, zum Reaktionsaustrag Carbonsäureanhydrid (V) zur Bindung des Wassers zuzuführen. Dieses kann beispielsweise direkt am Reaktorausgang oder in der Kolonne (z.B. Kolonnensumpf) zugegeben werden. Durch diese Maßnahme ist auch bei einer durch Wasser oder Methanol cokatalysierten Umesterung die Darstellung von wasserfreier Ameisensäure und von wasserfreiem Carbonsäureester (IV) möglich. Auch bei einem Einsatz von methanolhaltigem Ameisensäuremethylester als Ameisensäureester (I) sind somit wasserfreie Ameisensäure und wasserfreier Carbonsäureester (IV) problemlos darstellbar. Der beim Einsatz von Ameisensäuremethylester als Ameisensäureester (I) typische Restgehalt von etwa 2 bis 4 Gew.-% Methanol erweist sich in dessen Eigenschaft als Cokatalysator als Vorteil.

Die Umesterung kann sowohl in der Flüssigphase als auch in der Gasphase durchgeführt werden. Bei der Umesterung in der Gasphase setzt man bevorzugt heterogene Katalysatoren, wie beispielsweise die genannten Ionentauscher oder sauren Oxide ein. Bei der Umesterung in der Flüssigphase verwendet man homogene oder heterogene Katalysatoren. Bevorzugt wird die Umesterung in der Flüssigphase durchgeführt.

Im Allgemeinen führt man die Umesterung bei einer Temperatur von 20 bis 300°C und bevorzugt von 50 bis 180°C durch. Der Druck beträgt in der Regel 0,1 bis 5 MPa abs.

Die Umesterung kann in Gegenwart eines zusätzlichen inerten, polaren Lösungsmittels, erfolgen. Als inerte Lösungsmittel sind Lösungsmittel zu verstehen, welche unter den eingestellten Reaktionsbedingungen chemisch nicht mit den eingesetzten Verbindungen, d.h. den Edukten, den Produkten sowie den Katalysatoren, reagieren. Als geeignete Lösungsmittel seien beispielsweise Polyether genannt. Lösungsmittel werden in der Regel bei Umesterungen eingesetzt, bei denen Edukte und/oder Produkte zugegen sind, welche bei der gewünschten Temperatur, dem gewünschten Druck und den gewünschten Mengenverhältnissen der Edukte und Produkte im lösungsmittelfreien Reaktionsgemisch nur unzureichend löslich sind. Sind die Edukte und die Produkte unter den gewählten Bedingungen auch im lösungsmittelfreien Reaktionsgemisch löslich, so führt man die Umesterung bevorzugt ohne Zusatz eines Lösungsmittels aus.

Die Edukte Ameisensäureester (I) und Carbonsäure (II) werden im Allgemeinen in jeweils stöchiometrischer Menge zugegeben.

Durch zusätzliche Zugabe eines der beiden Edukte, beispielsweise als Vorlage vor Beginn der Reaktion, kann im Reaktionsgemisch gezielt ein astöchiometrisches Verhältnis der beiden Edukte eingestellt werden. So kann beispielsweise ein Edukt, welches gute Lösungseigenschaften besitzt, die Löslichkeit des anderen Edukts oder der Produkte verbessern. Ebenso ist es auch möglich, einen entsprechenden Überschuß eines der beiden Produkte im Reaktionsgemisch aufrecht zu erhalten.

Die Umesterung kann diskontinuierlich oder kontinuierlich erfolgen. Bevorzugt ist ein kontinuierliches Verfahren.

Für die Umesterung können beim erfindungsgemäßen Verfahren prinzipiell alle, für Umesterungsreaktionen bekannten Reaktionsapparate eingesetzt werden. Als geeignete Reaktionsapparate für die Umsetzung in der Flüssigphase seien beispielsweise Rührkesselreaktoren, Destillationskolonnen, Reaktivkolonnen und Membranreaktoren genannt. Um einen hohen Umsatz zu erreichen, ist es vorteilhaft, mindestens eines der beiden Produkte, bevorzugt alle beide, stetig aus dem Reaktionsgemisch zu entfernen. Beim Einsatz eines Rührkesselreaktors wird dies beispielsweise durch eine kontinuierliche Entnahme des Reaktionsgemisches, nachfolgender Trennung der beiden Produkte und Rückführung der beiden nicht-umgesetzten Edukte sowie gegebenenfalls des Katalysators erreicht. Beim Einsatz einer Destillationskolonne erfolgt die Umesterungsreaktion im Sumpf, wobei die leichter siedenden Komponenten destillativ getrennt und je nachdem, ob es sich um Edukt oder Produkt handelt, wieder rückgeführt oder abgeführt werden können Beim Einsatz einer Reaktivkolonne befindet sich der bevorzugt heterogene Katalysator im Trennbereich der Kolonne. Die leichter siedenden Komponenten werden hier ähnlich der beschriebenen Destillationskolonne destillativ getrennt und rückgeführt beziehungsweise abgeführt.

Als geeignete Reaktionsapparate für die Umsetzung in der Gasphase seien beispielsweise Strömungsrohre oder Schachtreaktoren genannt.

Die Trennung des Reaktionsgemisches kann auf verschiedene Weise erfolgen. Sie wird in der Regel durch die Eigenschaften der zu trennenden Edukte und Produkte bestimmt. Als Beispiele möglicher Trennverfahren seien die Destillation, die Kristallisation und die Extraktion genannt. Es sei darauf hingewiesen, dass auch Kombinationen verschiedener Trennverfahren, auch bei Voranschaltung einer Destillations- oder Reaktivkolonne zur Umesterung, möglich sind. Bevorzugt ist im Allgemeinen die destillative Trennung, welche gegebenenfalls auch als Destillation bei Unterdruck oder im Vakuum durchgeführt werden kann. Ist eine destillative Trennung nicht oder nur unter großem Aufwand möglich, beispielsweise bei höhersiedenden oder leicht zersetzlichen Komponenten, gewinnen die genannten alternativen Verfahren an Bedeutung. Bei Kenntnis der vorliegenden Edukte, Produkte und gegebenenfalls des Katalysators ist es für den Fachmann ohne Weiteres möglich, ein geeignetes Aufarbeitungskonzept zu entwickeln.

Ameisensäure (III) wird aufgrund ihrer guten Destillationseigenschaften in der Regel destillativ entfernt.

Bei der bevorzugten destillativen Trennung des erhaltenen Reaktionsgemisches werden in der Regel drei Destillationskolonnen beziehungsweise deren Äquivalente (z.B. eine Trennwandkolonne und eine Destillationskolonne) eingesetzt, um eine Trennung in vier Ströme zu erhalten. Der Ameisensäureester (I) enthaltende Strom wird im Allgemeinen zur Umesterung zurückgeführt, der Carbonsäureester (IV) enthaltende Strom wird teilweise oder ganz dem Carbonylierungsschritt (b) zugeführt, die Ameisensäure (III) wird aus dem System als Produkt abgeführt und der verbleibende, die Carbonsäure (II) enthaltende Strom wird im Allgemeinen ebenfalls zur Umesterung zurückgeführt.

Da bei der nachfolgenden Carbonylierung des Carbonsäureesters (IV) zum Carbonsäureanhydrid (V) in Gegenwart des Carbonylierungskatalysators gegebenenfalls noch vorhandener Ameisensäureester (I) zur entsprechenden Carbonsäure R¹-COOH isomerisiert wird, ist es in einer Variante mit vereinfachter destillativer Aufarbeitung unter Einsparung einer Destillationskolonne gegebenenfalls möglich, neben einen Ameisensäureester (I) enthaltenden Strom, einen Ameisensäure (III) enthaltenden Strom und einen Carbonsäure (II) enthaltenden Strom, als weiteren Strom einen Ameisensäureester (I) und Carbonsäureester (IV) enthaltenden Strom zu gewinnen und diesen dem Carbonylierungsschritt (b) zuzuführen. Dieser letztgenannte Strom kann beispielsweise als Seitenabzug der ersten Destillationskolonne gewonnen werden.

Beim erfindungsgemäßen Verfahren kann die gesamte Menge des erhaltenen Carbonsäureesters (IV) oder auch nur ein Teil davon dem Carbonylierungsschritt (b) zugeführt werden. Bei der letztgenannten Variante kann ein Teil des gebildeten Carbonsäureesters (IV) als Endprodukt erhalten werden. Der verbleibende Teil des Carbonsäureesters (IV) wird dem Carbonylierungsschritt (b) zugeführt.

In Schritt (b) carbonyliert man mindestens einen Teil, bevorzugt mindestens 5%, besonders bevorzugt mindestens 10% und ganz besonders bevorzugt mindestens 50% des in Schritt (a) gebildeten Carbonsäureesters (IV) in Gegenwart eines Katalysators zum entsprechenden Carbonsäureanhydrid (V).

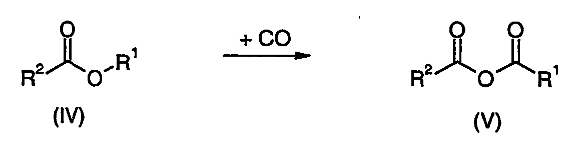

Beim erfindungsgemäßen Verfahren können in Schritt (b) die bekannten Verfahren zur Carbonylierung von Carbonsäureestern eingesetzt werden (siehe Ullmann's Encyclopedia of Industrial Chemistry, 6^{th} edition, 2000 electronic release, Chapter "ACETIC ANHYDRIDE AND MIXED FATTY ACID ANHYDRIDES - Acetic Anhydride - Production" und die untenstehenden Zitate).

Als Katalysatoren können im Allgemeinen Metalle der 8. bis 10. Gruppe des Periodensystems sowie deren Verbindungen in Gegenwart von Halogeniden sowie organischen Halogenverbindungen eingesetzt. Als bevorzugte Katalysatormetalle seien Rhodium, Iridium, Palladium, Nickel und Cobalt, insbesondere Rhodium, genannt (EP-A 0 677 505). Als Halogenide beziehungsweise organische Halogenverbindungen werden in der Regel lodverbindungen eingesetzt. Bevorzugt ist die Zugabe von Alkali- und Erdalkaliiodiden (US 5,003,104, US 4,559,183), Iodwasserstoffsäure, Iod, lodalkane, insbesondere Iodmethan (Methyliodid) (GB-A 2,333,773, DE-OS 24 41 502) oder substituiertes Azoliumiodid (EP-A 0 479 463). Die Katalysatormetalle sind in der Regel durch Liganden stabilisiert. Als geeignete Liganden werden bevorzugt Stickstoff- und Phosphorverbindungen, wie beispielsweise N-haltige heterocyclische Verbindungen (DE-OS 28 36 084), Amine, Amide (DE-OS 28 44 371) oder Phosphine (US 5,003,104, EP-A 0 336 216) eingesetzt. Die Katalysatorsysteme können ferner noch Promotormetalle, wie beispielsweise Chrom im System Nickel/Chrom (US 4,002,678), Ruthenium im System Iridium/Ruthenium (GB-A 2,333,773) oder Cobalt im System Ruthenium/Cobalt (US 4,519,956) enthalten. Als bevorzugte Katalysatorsysteme seien Systeme welche Rhodium und/oder Iridium, Methyliodid, Stickstoff- und/oder Phosphorenthaltende Liganden sowie gegebenenfalls Promotoren, wie beispielsweise Lithium oder Chrom, enthalten, genannt. Besonders bevorzugt ist der Einsatz eines Katalysators auf Basis Rhodiumtriiodid, Lithiumiodid und Iodmethan, wie beispielsweise in US 4,374,070 beschrieben.

Der Katalysator kann ungeträgert als sogenannter Homogenkatalysator oder geträgert als sogenannter Heterogenkatalysator eingesetzt werden. Als geeignete Trägermaterialien seien beispielhaft anorganische Oxide, wie etwa Siliziumdioxid oder Aluminiumoxid (EP-A 0 336 216), oder Polymere, wie etwa Ionentauscher (J6 2135 445) oder Harze (JP 09124 544) genannt.

Die Carbonylierung kann in Gegenwart von Wasserstoff (US 5,003,104, GB-A 2 333 773, US 4,333,885, WO 82/01704) oder in Abwesenheit von Wasserstoff (A.C. Marr et al., Inorg. Chem. Comm. 3, 2000, Seite 617 bis 619) durchgeführt werden. Im Allgemeinen ist es vorteilhaft, die Carbonylierung in Gegenwart von Wasserstoff durchzuführen, wobei man in der Regel Wasserstoffkonzentrationen vom ppm-Bereich bis hin zu 15 Vol.-% und bevorzugt von 1 bis 10 Vol.-%, bezogen auf den zugeführten gasförmigen Eduktstrom, wählt.

Die Carbonylierung kann sowohl in der Gasphase (EP-A 0 336 216) als auch in der Flüssigphase durchgeführt werden. Bei einer Durchführung in der Gasphase setzt man im Allgemeinen geträgerte Katalysatoren ein. Bevorzugt ist beim erfindungsgemäßen Verfahren die Carbonylierung in der Flüssigphase.

Die Carbonylierung in der Gasphase führt man im Allgemeinen bei einer Temperatur von 130 bis 400°C, bevorzugt von 150 bis 280°C und einem Druck von 0,1 bis 15 MPa abs, bevorzugt 0,5 bis 3 MPa abs durch. Die Carbonylierung in der Flüssigphase führt man im Allgemeinen bei einer Temperatur von 100 bis 300°C, bevorzugt von 170 bis 200°C und einem Druck von 0,1 bis 15 MPa abs, bevorzugt 1 bis 8 MPa abs durch.

Bei der bevorzugten Carbonylierung in der Flüssigphase und dem Einsatz eines Homogenkatalysators setzt man in der Regel eine Katalysatorkonzentration im Bereich von 0,01 bis 1 Gew.-% bezogen auf die Reaktionslösung ein.

Die Carbonylierung kann in Gegenwart eines zusätzlichen inerten Lösungsmittels erfolgen. Als inerte Lösungsmittel sind Lösungsmittel zu verstehen, welche unter den eingestellten Reaktionsbedingungen chemisch nicht mit den eingesetzten Verbindurigen, d.h. den Edukten, den Produkten sowie den Katalysatoren, reagieren. Geeignete inerte Lösungsmittel sind beispielsweise aromatische und aliphatische Kohlenwasserstoffe sowie Carbonsäuren oder deren Ester. Bevorzugt werden Lösungsmittel bei Carbonylierungen eingesetzt, bei denen das Edukt und/oder das Produkt bei der gewünschten Temperatur und/oder dem gewünschten Druck im lösungsmittelfreien Reaktionsgemisch nur unzureichend löslich sind. Sind die Edukte und die Produkte unter den gewählten Bedingungen auch im lösungsmittelfreien Reaktionsgemisch löslich, so führt man die Umesterung bevorzugt ohne Zusatz eines Lösungsmittels aus.

Die Carbonylierung kann diskontinuierlich oder kontinuierlich erfolgen. Bevorzugt ist ein kontinuierliches Verfahren.

Für die Carbonylierung können beim erfindungsgemäßen Verfahren prinzipiell alle, für Carbonylierungsreaktionen bekannten Reaktionsapparate eingesetzt werden. Die Carbonylierung in der Gasphase führt man im Allgemeinen in einem Strömungsrohr oder Schachtreaktor durch. Als geeignete Reaktionsapparate für die bevorzugte Carbonylierung in der Flüssigphase seien beispielsweise Rührkesselreaktoren, Strahlschlaufenreaktoren und Blasensäulen genannt. Im Folgenden ist deren Einsatz in einem kontinuierlichen Verfahren kurz beschrieben.

Beim Einsatz der genannten Reaktionsapparate führt man in der Regel die gewünschten Mengen an Carbonsäureester (IV) und Kohlenmonoxid kontinuierlich zur Reaktionslösung, welche insbesondere das Carbonsäureanhydrid (V), den Carbonylierungskatalysator und gegebenenfalls ein zusätzliches Lösungsmittel enthält, unter intensiver Durchmischung zu. Die gebildete Carbonylierungswärme kann beispielsweise durch innenliegende Wärmetauscher, durch Kühlung der Wandung des Reaktionsapparats und/oder durch kontinuierliche Entnahme der heißen Reaktionslösung, außenliegende Kühlung und Rückführung entzogen werden. Beim Einsatz eines Strahlschlaufenreaktors oder einer Blasensäule ist zur Sicherstellung der Durchmischung ein externer Kreislauf erforderlich. Die Produktabfuhr erfolgt durch kontinuierliche Entnahme und anschließende Abtrennung des Carbonylierungskatalysators in einer geeigneten Abtrennvorrichtung. Als geeignete Abtrennvorrichtung sei beispielsweise ein sogenannter Flash-Verdampfer genannt, bei dem das Carbonsäureanhydrid (V) durch Druckentspannung verdampft. Die verbleibende Lösung, welche den Carbonylierungskatalysator enthält, wird dem Reaktionsapparat wieder zugeführt. Durch geeignete Temperatur- und Druckführung ist es gegebenenfalls auch möglich, das gebildete Carbonsäureanhydrid durch Verdampfung aus der Reaktionslösung kontinuierlich abzuziehen (DE-OS 30 24 353). Das verdampfte Carbonsäureanhydrid (V) kann je nach Erfordernis einer Aufarbeitungsstufe oder einer Folgestufe zur weiteren Umsatzung zugeführt werden. Bei höhersiedenden Carbonsäureanhydriden (V), bei denen die beschriebene Flashverdampfung aufgrund deren geringer Flüchtigkeit nicht möglich ist, ist der Reaktionsaustrag durch andere Maßnahmen, beispielsweise destillativ unter Unterdruck, durch Kristallisation oder Extraktion aufzuarbeiten.

Die beim erfindungsgemäßen Verfahren zu wählenden Verfahrensparameter und Maßnahmen sind unter anderem von der Natur des eingesetzten Carbonsäureesters (IV), des gebildeten Carbonsäureanhydrids (V) und dem ausgewählten Katalysatorsystem abhängig und mit dem üblichen Fachkönnen zu ermitteln.

Abhängig von den gewählten Edukten Ameisensäureester (I) und Carbonsäure (II) wird durch die Carbonylierung in Schritt (b) ein symmetrisches oder asymmetrisches Carbonsäureanhydrid gebildet, d.h. die Reste R¹ und R² können identisch oder verschieden sein.

Des Weiteren ist es möglich, zu dem zu carbonylierenden Carbonsäureester (IV) einen Alkohol R¹-OH oder R²-OH zuzuführen. Der Alkohol wird dabei zur entsprechenden Carbonsäure R¹-COOH beziehungsweise R²-COOH (II) umgesetzt. Durch eine derartige Zufuhr ist es möglich, das Verhältnis zwischen den Carbonylierungsprodukten R²-COOH (II), Carbonsäureanhydrid (V) und R¹-COOH zur Ameisensäure (I) zu erhöhen. So führt beispielsweise die zusätzliche Zufuhr von Methanol bei der Carbonylierung von Essigsäuremethylester zur Bildung von Essigsäure neben Essigsäureanhydrid aus der Carbonylierung des Essigsäuremethylesters. Ferner ist es auch möglich, dem zu carbonylierenden Carbonsäureester (IV) als weitere Komponente zusätzlich Wasser, Carbonsäureester (IV), Ameisensäureester (I) oder Ether der allgemeinen Formeln R¹-O-R¹, R¹-O-R² oder R²-O-R² zuzuführen.

Beim erfindungsgemäßen Verfahren kann die gesamte Menge des erhaltenen Carbonsäureanhydrids (V) oder auch nur ein Teil davon dem Umanhydridisierungsschritt (c) zugeführt werden. Bei der letztgenannten Variante kann ein Teil des gebildeten Carbonsäureanhydrids (V) als Endprodukt erhalten werden. Der verbleibende Teil des Carbonsäureanhydrids (V) wird dem Umanhydridisierungsschritt (c) zugeführt.

In Schritt (c) umanhydridisiert man mindestens einen Teil, bevorzugt mindestens 5%, besonders bevorzugt mindestens 10% und ganz besonders bevorzugt mindestens 50% des in Schritt (b) gebildeten Carbonsäureanhydrids (V) durch Umsetzung mit einer Carbonsäure (VI).

Die einzusetzende Carbonsäure besitzt die allgemeine Formel (VI) 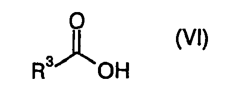 in der der Rest R³ für einen Kohlenstoff enthaltenden organischen Rest steht. Unter einem Kohlenstoff enthaltenden organischen Rest ist bevorzugt ein unsubstituierter oder substituierter, aliphatischer, aromatischer oder araliphatischer Rest mit 1 bis 12 Kohlenstoffatomen, welcher ein oder mehrere Heteroatome, wie etwa Sauerstoff, Stickstoff oder Schwefel, wie beispielsweise -O-, -S-, -NR-, -CO- und/oder -N= in aliphatischen oder aromatischen Systemen, enthalten kann, und/oder durch eine oder mehrere funktionelle Gruppen, welche beispielsweise Sauerstoff, Stickstoff, Schwefel und/oder Halogen enthalten können, wie beispielsweise durch Fluor, Chlor, Brom, Iod und/oder eine Cyanogruppe, substituiert sein kann, zu verstehen.

Bei der genannten Umanhydridisierung in Schritt (c) handelt es sich um eine Gleichgewichtsreaktion. Gemäß dem folgenden Reaktionsschema werden die Edukte Carbonsäureanhydrid (V) und Carbonsäure (VI) umgesetzt zu den Produkten Carbonsäure (II), Carbonsäure (IIa) und Carbonsäureanhydrid (VII).

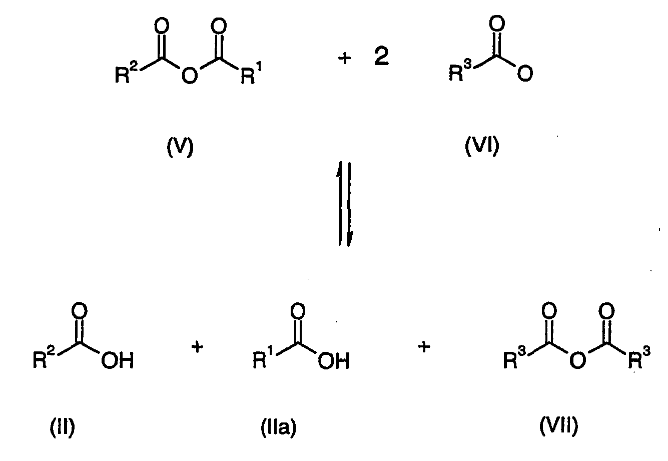

Beim erfindungsgemäßen Verfahren können in Schritt (c) die bekannten Verfahren zur Umanhydridisierung eingesetzt werden. Geeignete Verfahren sind beispielsweise in DE-A 35 10 035, EP-A 0 231 689, DE-A 36 44 222 und EP-A 1 231 201 beschrieben.

Um die Reaktionsgeschwindigkeit zu erhöhen ist es im Allgemeinen vorteilhaft, die Umanhydridisierung in Gegenwart von Katalysatoren durchzuführen. Als Katalysatoren eignen sich insbesondere saure oder basische Substanzen sowie geeignete Metallionen.

Werden saure Substanzen als Katalysatoren eingesetzt, so können diese unter den Reaktionsbedingungen prinzipiell fest, flüssig oder gasförmig vorliegen. Als geeignete feste saure oder basische Katalysatoren seien beispielsweise saure oder basische Ionentauscher und saure oder basische Oxide, wie etwa Zeolithe, Aluminosilikate, SiO₂/TiO₂ oder Übergangsmetalloxide genannt. Als geeignete flüssige oder gasförmige saure Katalysatoren seien organische oder anorganische Säuren, welche einen niedrigeren pKa-Wert als die Carbonsäure (VI) und die Carbonsäure (II) aufweisen, genannt. Als organische oder anorganische Säuren werden vorzugsweise Schwefelsäure, aliphatische oder aromatische Sulfonsäuren oder Phosphorsäure eingesetzt. Die Menge an organischer oder anorganischer Säure liegt zweckmäßigerweise bei 0,01 bis 2 Mol-%, bevorzugt bei 0,1 bis 2 Mol-%, bezogen auf die eingesetzte Carbonsäure (VI).

Werden Metallionen als Katalysatoren eingesetzt, so handelt es sich bevorzugt um Metallionen der 1. bis 13. Gruppe des Periodensystems. Als bevorzugt sind die Metallionen von Cobalt, Chrom, Nickel, Mangan, Eisen, Lithium, Natrium, Kalium, Magnesium, Barium, Kalzium, Kupfer, Zink, Zirkon, Titan, Lanthan, Scandium, Wolfram, Cer, Molybdän, Thorium, Yttrium, Niob, Tantal, Hafnium, Rhenium, Aluminium und Vanadium genannt. Die Konzentration an Metallionen im Reaktionsansatz liegt zweckmäßigerweise bei 5 bis 1000 Gew.-ppm, bevorzugt 50 bis 500 Gew.-ppm.

Die Umanhydridisierung kann in der Flüssigphase als auch in der Gasphase durchgeführt werden. Bei der Umanhydridisierung in der Gasphase setzt man bevorzugt heterogene Katalysatoren, wie beispielsweise die genannten Ionentauscher oder sauren Oxide ein. Bei der Umanhydridisierung in der Flüssigphase verwendet man als Katalysatoren bevorzugt die genannten organischen oder anorganischen Säuren oder Metallionen. Bevorzugt führt man die Umanhydridisierung in der Flüssigphase oder Flüssig/Gasphase durch.

Im Allgemeinen führt man die Umanhydridisierung bei einer Temperatur von 20 bis 300°C und bevorzugt von 30 bis 200°C durch. Der Druck beträgt in der Regel 0,001 bis 5 MPa abs und bevorzugt 0,01 bis 0,5 MPa abs.

Die Umanhydridisierung kann in Gegenwart eines zusätzlichen inerten, polaren Lösungsmittels erfolgen. Als inerte Lösungsmittel sind Lösungsmittel zu verstehen, welche unter den eingestellten Reaktionsbedingungen chemisch nicht mit den eingesetzten Verbindungen, d.h. den Edukten, den Produkten sowie den Katalysatoren, reagieren. Als geeignete Lösungsmittel seien beispielsweise aromatische Kohlenwasserstoffe oder Polyether genannt. Lösungsmittel werden in der Regel bei Umesterungen eingesetzt, bei denen Edukte und/oder Produkte zugegen sind, welche bei der gewünschten Temperatur, dem gewünschten Druck und den gewünschten Mengenverhältnissen der Edukte und Produkte im lösungsmittelfreien Reaktionsgemisch nur unzureichend löslich sind. Sind die Edukte und die Produkte unter den gewählten Bedingungen auch im lösungsmittelfreien Reaktionsgemisch löslich, so führt man die Umanhydridisierung bevorzugt ohne Zusatz eines Lösungsmittels aus.

Die Edukte Carbonsäureanhydrid (V) und Carbonsäure (VI) werden im Allgemeinen in der jeweils stöchiometrisch erforderlichen Menge zugegeben. Gegegebenenfalls ist von Vorteil, einen Überschuß an Carbonsäureanhydrid (V) einzusetzen, um das Gleichgewicht in Richtung des gewünschten Carbonsäureanhydrids (VII) zu verschieben und einen nach außen hin vollständigen Umsatz der eingesetzten Carbonsäure (VI) zu erreichen. Dieser Überschuß an Carbonsäureanhydrid (V) beträgt vorteilhafterweise bis zu 0,5 Mol pro Mol Carbonsäure (VI).

Die Umanhydridisierung kann diskontinuierlich oder kontinuierlich erfolgen. Bevorzugt ist ein kontinuierliches Verfahren unter kontinuierlicher Zufuhr der Edukte Carbonsäureanhydrid (V) und Carbonsäure (VI) sowie unter kontinuierlicher Abfuhr des Reaktionsgemisches zur weiteren Aufarbeitung beziehungsweise unter kontinuierlicher Abfuhr des gewünschten Produkts Carbonsäureanhydrid (VII) sowie der gebildeten Carbonsäuren (II) und (IIa) und gegebenenfalls des überschüssigen Carbonsäureanhydrids (V).

Für die Umanhydridisierung können beim erfindungsgemäßen Verfahren prinzipiell alle, für Umanhydridisierungsreaktionen bekannten Reaktionsapparate eingesetzt werden. Als geeignete Reaktionsapparate für die Umsetzung in der Flüssigphase seien beispielsweise Rührkesselreaktoren, Destillationskolonnen, Reaktivkolonnen und Membranreaktoren genannt. Um einen hohen Umsatz zu erreichen, ist es vorteilhaft, mindestens eines der beiden Produkte, bevorzugt alle Produkte, das heißt das Carbonsäureanhydrid (VII) und die Carbonsäuren (II) und (IIa), stetig aus dem Reaktionssystem zu entfernen.

Beim Einsatz eines Rührkesselreaktors wird dies beispielsweise durch eine kontinuierliche Entnahme des Reaktionsgemisches, nachfolgender Abtrennung der Produkte und Rückführung der nicht-umgesetzten Edukte sowie gegebenenfalls des Katalysators erreicht. Die nachfolgende Abtrennung erfolgt im Allgemeinen durch den Einsatz einer oder mehrerer Destillationskolonnen. Die Ausarbeitung eines, für das konkrete System geeigneten Trennverfahrens ist mit dem üblichen Fachkönnen möglich.

Bevorzugt ist die Durchführung der Umanhydridisierungsreaktion in einer Destillations- oder Reaktivkolonne. Ein für das erfindungsgemäße Verfahren geeignetes Verfahren ist beispielsweise in DE-A 3510 035 beschrieben. Bei der Umanhydridisierung in einer Destillations- oder Reaktivkolonne erfolgt die Reaktion bevorzugt im mittleren Bereich der Kolonne. Das Carbonsäureanhydrid (V) und die Carbonsäure (VI) werden dabei seitiich im Mittelteil der Kolonne zugeführt. Im Allgemeinen und insbesondere beim Einsatz von Essigsäureanhydrid als Carbonsäureanhydrid (V) ist die gebildete Carbonsäure (II) und (IIa), welche beim Einsatz von Essigsäureanhydrid identisch ist und es sich um Essigsäure handelt, die bei der niedrigsten Temperatur siedende Komponente. Sie wird daher in der Regel kontinuierlich über Kopf abgeführt. Das gebildete Carbonsäureanhydrid (VII) ist im Allgemeinen die bei der höchsten Temperatur siedende Komponente und wird in der Regel kontinuierlich über den Sumpf entnommen. Um in der Kolonne eine entsprechende Reaktionszone zu ermöglichen, ist es besonders vorteilhaft, das Carbonsäureanhydrid (V) unterhalb der Carbonsäure (VI) zuzugeben, so dass sich die Reaktionspartner im Gegenstromprinzip begegnen. Die Zugabe im Gegenstromprizip führt des Weiteren auch zu einer Erhöhung des Umsatzes, da beispielsweise im unteren Bereich der Kolonne eine hohe Konzentration an Carbonsäureanhydrid (V) vorliegt, welche in Verbindung mit einer eher niedrigeren Konzentration an Carbonsäure (VI) das Gleichgewicht in Richtung des gewünschten Produkts Carbonsäureanhydrid (VII) verschiebt. Ferner ist es aber auch möglich, das Carbonsäureanhydrid (V) und die Carbonsäure (VI) zusammen an einer Stelle in die Kolonne zu geben. Dies mag beispielsweise von Vorteil sein, wenn beide Edukte identische oder sehr ähnliche Siedepunkte aufweisen. Wird ein heterogener Katalysator eingesetzt, so befindet sich dieser bevorzugt in Form immobilisierter Packungen oder Beschichtungen innerhalb des Kolonnenbereichs. Werden homogene Katalysatoren eingesetzt, so werden diese als weitere Komponente, in der Regel ebenfalls kontinuierlich, der Kolonne zugeführt. Metallionen als homogene Katalysatoren werden in der Regel im oberen Bereich der Kolonne zugeführt und über den Sumpf ausgetragen, vom Sumpfprodukt abgetrennt und in der Regel rückgeführt. So werden beispielsweise flüssige Säuren, welche über das Sumpfprodukt abgeführt werden, bevorzugt im oberen Bereich der Kolonne zugeführt. Analog der Beschreibung zum Einsatz von Metallionen, werden die mit dem Sumpf ausgetragenen organischen oder anorganischen Säuren ebenfalls vom Carbonsäureanhydrid (VII) abgetrennt und im Allgemeinen zurückgeführt. Organische oder anorganische Säuren werden in der Regel in dem zur Entnahme eher entgegengesetzten Bereich zugeführt, so dass diese in der Kolonne verteilt vorliegen. So werden beispielsweise höhersiedende organische oder anorganische Säuren, welche entsprechend den in der Kolonne vorliegenden Bedingungen über den Sumpf entnommen werden, bevorzugt im oberen Bereich zugegeben.

Entsprechend der obigen Ausführung ist es beim erfindungsgemäßen Verfahren besonders vorteilhaft, die Umanhydridisierung in Schritt (c) in einer kontinuierlich betriebenen Destillationskolonne durchzuführen und die gebildeten Reaktionsprodukte Carbonsäure (II) und Carbonsäureanhydrid (VII) kontinuierlich abzuführen.

Als geeignete Reaktionsapparate für die Umsetzung in der Gasphase seien beispielsweise Strömungsrohre oder Schachtreaktoren genannt.

Die weitere und gegebenenfalls erforderliche Aufreinigung der erhaltenen Produkte kann bei Kenntnis der Edukte, Produkte und gegebenenfalls des Katalysators mit dem üblichen Fachwissen entwickelt werden.

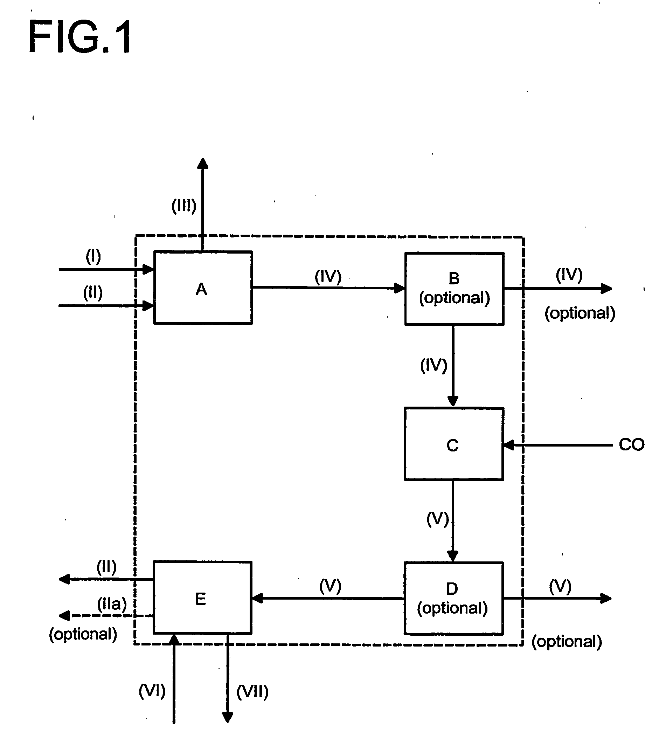

Abbildung 1 zeigt ein Blockdiagramm des erfindungsgemäßen Verfahrens. Ameisensäureester (I) und Carbonsäure (II) werden in Block "A" (Umesterung/Trennung) unter Bildung von Ameisensäure (III) und Carbonsäureester (IV) umgesetzt. Die abgetrennte Ameisensäure (III) wird als Endprodukt abgeführt. Der abgetrennte Carbonsäureester (IV) wird über einen optional vorhandenen Block "B" (Ausschleusung Carbonsäureester), bei dem gegebenenfalls ein Teil des gebildeten Carbonsäureesters (IV) als Endprodukt ausgeschleust werden kann, dem Block "C" (Carbonylierung) zugeführt. Unter Zufuhr von Kohlenmonoxid wird Carbonsäureanhydrid (V) gebildet. Das Carbonsäureanhydrid (V) wird über einen optional vorhandenen Block "D" (Ausschleusung Carbonsäureanhydrid), bei dem gegebenenfalls ein Teil des gebildeten Carbonsäureanhydrids (V) als Endprodukt ausgeschleust werden kann, dem Block "E" (Umanhydridisierung) zugeführt. Dort wird unter Zufuhr von Carbonsäure (VI) Carbonsäureanhydrid (VII) und Carbonsäure (II) sowie für den Fall des Einsatzes eines asymmetrischen Carbonsäureanhydrids (V) auch Carbonsäure (IIa) gebildet und als Produkte ausgeschleust.

In einer bevorzugten Ausführungsform des erfindungsgemäßen Verfahrens setzt man mindestens einen Teil der in Schritt (c) gebildeten Carbonsäure (II) zu Schritt (a) zurück. Für das erfindungsgemäße Verfahren ist es dabei besonders vorteilhaft, insgesamt etwa die Menge an Carbonsäure (II) zu Schritt zurückzuführen, welche dort für die Aufrechterhaltung des Kreislaufs nötig ist. Um die Aufpegelung unerwünschter Nebenprodukte zu vermeiden, ist es gegebenenfalls vorteilhaft, geringfügig weniger Carbonsäure (II) als erforderlich zu Schritt (a) zurückzuführen und die Differenz durch die Zufuhr frischer Carbonsäure (II) zu decken.

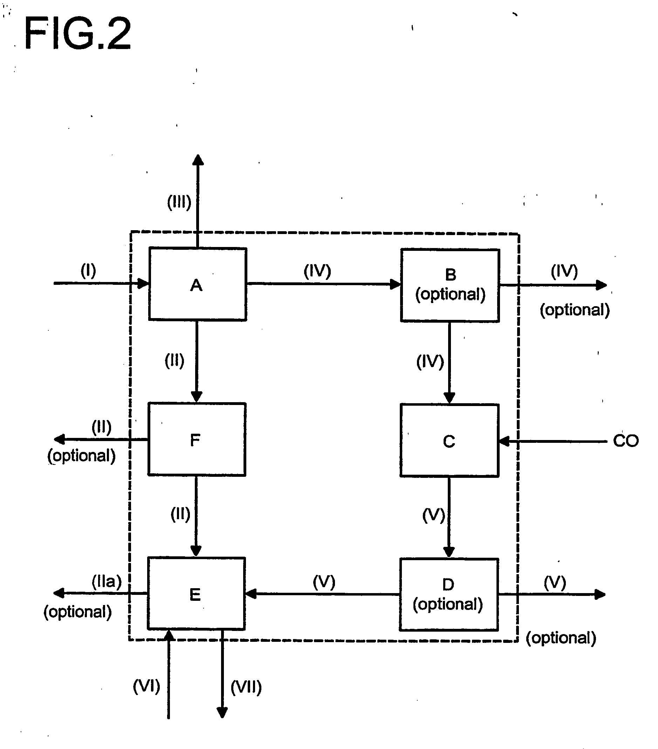

Abbildung 2 zeigt ein Blockdiagramm des bevorzugten erfindungsgemäßen Verfahrens. Für die Blöcke "A" bis "E" sei auf die Beschreibung des Blockdiagramms von Abbildung 1 verwiesen. Bei diesem bevorzugten Verfahren wird nun die aus Block "E" (Umanhydridisierung) stammende Carbonsäure (II) über einen optional vorhandenen Block "F" (Ausschleusung Carbonsäure), bei dem gegebenenfalls ein Teil der gebildeten Carbonsäure (II) als Endprodukt ausgeschleust werden kann, dem Block "A" (Umesterung/Trennung) zugeführt.

Beim erfindungsgemäßen Verfahren setzt man bevorzugt einen Ameisensäureester (I) 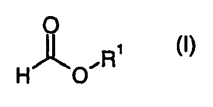 ein, bei dem der Rest R¹ für
- einen unsubstituierten oder substituierten, unverzweigten oder verzweigten, acyclischen oder cyclischen C₁- bis C₁₂-Alkylrest, wie beispielsweise Methyl, Ethyl, 1-Propyl, 2-Propyl, 1-Butyl, 2-Butyl, 2-Methyl-1-propyl, 2-Methyl-2-propyl, 1-Pentyl, 2-Pentyl, 3-Pentyl, 3-Methyl-2-butyl, 2-Methyl-2-butyl, Hexyl, Heptyl, 2-Ethyl-1-pentyl, Octyl, 2,4,4-Trimethyl-1-pentyl, Nonyl, 1,1-Dimethyl-1-heptyl, Decyl, Undecyl, Dodecyl, Phenylmethyl, 2-Phenylethyl, 3-Phenylpropyl, Cyclopentyl, Cyclopentylmethyl, 2-Cyclopentylethyl, 3-Cyclopentylpropyl, Cyclohexyl, Cyclohexylmethyl, 2-Cyclohexylethyl oder 3-Cyclohexylpropyl; oder
- einen unsubstituierten oder substituierten, unverzweigten oder verzweigten, a-cyclischen oder cyclischen C₂- bis C₁₂-Alkenylrest, wie beispielsweise Vinyl (Ethenyl), 1-Propenyl, 2-Propenyl, 1-Methylvinyl, 3-Butenyl, cis-2-Butenyl, trans-2-Butenyl, cis-1-Butenyl, trans-1-Butenyl, Pentenyl, Hexenyl, Heptenyl, Octenyl, Nonenyl, Decenyl, 3-Cyclopentenyl, 2-Cyclohexenyl, 3-Cyclohexenyl oder 2,5-Cyclohexadienyl;
steht.

Besonders bevorzugt setzt man einen Ameisensäureester (I) ein, bei dem der Rest R¹ für einen unsubstituierten, unverzweigten oder verzweigten, acyclischen C₁- bis C₆-Alkylrest, konkret Methyl, Ethyl, 1-Propyl, 2-Propyl, 1-Butyl, 2-Butyl, 2-Methyl-1-propyl, 2-Methyl-2-propyl, 1-Pentyl und 1-Hexyl steht. Ganz besonders bevorzugt setzt man Ameisensäuremethylester, Ameisensäureethylester, Ameisensäurepropylester und Ameisensäurebutylester und insbesondere Ameisensäuremethylester ein.

Beim erfindungsgemäßen Verfahren setzt man bevorzugt eine Carbonsäure (II) 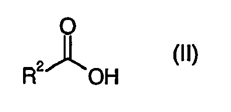 ein, bei der der Rest R² für
- einen unsubstituierten oder substituierten, unverzweigten oder verzweigten, acyclischen oder cyclischen C₁- bis C₁₂-Alkylrest, wie beispielsweise Methyl, Ethyl, 1-Propyl, 2-Propyl, 1-Butyl, 2-Butyl, 2-Methyl-1-propyl, 2-Methyl-2-propyl, 1-Pentyl, 2-Pentyl, 3-Pentyl, 3-Methyl-2-butyl, 2-Methyl-2-butyl, Hexyl, Heptyl, 2-Ethyl-1-pentyl, Octyl, 2,4,4-Trimethyl-1-pentyl, Nonyl, 1,1-Dimethyl-1-heptyl, Decyl, Undecyl, Dodecyl, Phenylmethyl, 2-Phenylethyl, 3-Phenylpropyl, Cyclopentyl, Cyclopentylmethyl, 2-Cyclopentylethyl, 3-Cyclopentylpropyl, Cyclohexyl, Cyclohexylmethyl, 2-Cyclohexylethyl, 3-Cyclohexylpropyl, Chlormethyl, Dichlormethyl, Trichlormethyl; oder
- einen unsubstituierten oder substituierten, unverzweigten oder verzweigten, acyclischen oder cyclischen C₂- bis C₁₂-Alkenylrest, wie beispielsweise Vinyl (Ethenyl), 1-Propenyl, 2-Propenyl, 1-Methylvinyl, 3-Butenyl, cis-2-Butenyl, trans-2-Butenyl, cis-1-Butenyl, trans-1-Butenyl, Pentenyl, Hexenyl, Heptenyl, Octenyl, Nonenyl, Decenyl, 3-Cyclopentenyl, 2-Cyclohexenyl, 3-Cyclohexenyl oder 2,5-Cyclohexadienyl;
   steht.
   Besonders bevorzugt setzt man eine Carbonsäure (II) ein, bei der der Rest R² für
- einen unsubstituierten oder substituierten, unverzweigten oder verzweigten, acyclischen C₁- bis C₆-Alkylrest, konkret Methyl, Ethyl, 1-Propyl, 2-Propyl, 1-Butyl, 2-Butyl, 2-Methyl-1-propyl, 2-Methyl-2-propyl, 1-Pentyl, 2-Pentyl, 3-Pentyl, 3-Methyl-2-butyl, 2-Methyl-2-butyl, Hexyl, Chlormethyl, Dichlormethyl oder Trichlormethyl; oder
- einen unsubstituierten, unverzweigten oder verzweigten, acyclischen C₂- bis C₆-Alkenylrest, wie beispielsweise Vinyl (Ethenyl), 1-Propenyl, 2-Propenyl, 1-Methylvinyl, 3-Butenyl, cis-2-Butenyl, trans-2-Butenyl, cis-1-Butenyl, trans-1-Butenyl, Pentenyl oder Hexenyl;
steht. Ganz besonders bevorzugt setzt man Essigsäure und Propionsäure, insbesondere Essigsäure ein.

Beim erfindungsgemäßen Verfahren setzt man bevorzugt eine Carbonsäure (VI) 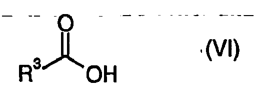 ein, bei der der Rest R³ für
- einen unsubstituierten oder substituierten, unverzweigten oder verzweigten, acyclischen oder cyclischen C₂- bis C₃₀-Alkylrest, wie beispielsweise Ethyl, 1-Propyl, 2-Propyl, 1-Butyl, 2-Butyl, 2-Methyl-1-propyl, 2-Methyl-2-propyl, 1-Pentyl, 2-Pentyl, 3-Pentyl, 3-Methyl-2-butyl, 2-Methyl-2-butyl, Hexyl, Heptyl, 2-Ethyl-1-pentyl, Octyl, 2,4,4-Trimethyl-1-pentyl, Nonyl, 1,1-Dimethyl-1-heptyl, Decyl, Undecyl, Dodecyl, Tridecyl, Tetradecyl, Pentadecyl, Hexadecyl, Heptadecyl, Octadecyl, Nonadecyl, Icosyl, Henicosyl, Docosyl, Tricosyl, Tetracosyl, Pentacosyl, Hexacosyl, Heptacosy, Octacosyl, Nonacosyl, Triacontyl, Phenylmethyl, 2-Phenylethyl, 3-Phenylpropyl, Cyclopentyl, 2-Carboxy-cyclopentyl, Cyclopentylmethyl, 2-Cyclopentylethyl, 3-Cyclopentylpropyl, Cyclohexyl, 2-Carboxycyclohexyl, Cyclohexylmethyl, 2-Cyclohexylethyl, 3-Cyclohexylpropyl, Chlormethyl, Dichlormethyl oder Trichlormethyl;
- einen unsubstituierten oder substituierten, unverzweigten oder verzweigten, acyclischen oder cyclischen C₂- bis C₃₀-Alkenylrest, C₂- bis C₃₀-Alkadienylrest oder C₂- bis C₃₀-Alkatrienylrest, wie beispielsweise Vinyl (Ethenyl), 1-Propenyl, 2-Propenyl, 1-Methylvinyl, 3-Butenyl, cis-2-Butenyl, trans-2-Butenyl, cis-1-Butenyl, trans-1-Butenyl, Pentenyl, Hexenyl, Heptenyl, Octenyl, Nonenyl, Decenyl, cis-8-Heptadecenyl, trans-8-Heptadecenyl, cis,cis-8,11-Heptadecadienyl, cis,cis,cis-8,11,14-Heptadecatrienyl, 3-Cyclopentenyl, 2-Cyclohexenyl, 3-Cyclohexenyl oder 2,5-Cyclohexadienyl;
- einen unsubstituierten oder mit einem oder mehreren C₁- bis C₄-Alkylresten substituierten C₆- bis C₂₀-Aryl- oder C₃- bis C₂₀-Heteroaryl-Rest, wie beispielsweise Phenyl, 2-Carboxy-phenyl, 2,4,5-Tricarboxy-phenyl, 2-Methylphenyl (o-Tolyl), 3-Methylphenyl (m-Tolyl), 4-Methylphenyl (p-Tolyl), 2,6-Dimethylphenyl, 2,4-Dimethylphenyl, 2,4,6-Trimethylphenyl, 2-Methoxyphenyl, 3-Methoxyphenyl, 4-Methoxyphenyl, 1-Naphthyl, 2-Napthyl, 2-Carboxy-1-napththyl, 3-Carboxy-2-naphthyl, 3,6,7-Tricarboxy-2-naphthyl, 8-Carboxy-1-naphthyl, 4,5,8-Tricarboxy-1-naphthyl, 2-Carboxy-1-anthracenyl, 3-Carboxy-2-anthracenyl, 3,6,7-Tricarboxy-2-anthracenyl, 4,9,10-Tricarboxy-3-perylen oder 4,3',4'-Tricarboxy-3-benzophenonyl;
steht.

Besonders bevorzugt setzt man als Carbonsäure (VI) Propionsäure, Buttersäure, Pentansäure, Hexansäure, 2-Ethyl-hexansäure, Acrylsäure, Methacrylsäure, Phthalsäure, Benzol-1,2,4,5-tetracarbonsäure (Pyromellitsäure), Benzophenon-3,3',4,4'-tetracarbonsäure, Naphthalin-2,3,6,7-tetracarbonsäure oder Napthalin-1,4,5,8-tetracarbonsäure ein.

Besonders bevorzugt stellt man beim erfindungsgemäßen Verfahren als Carbonsäureanhydrid (VII) Propionsäureanhydrid, Buttersäureanhydrid, Acrylsäureanhydrid, Methacrylsäureanhydrid und/oder Benzol-1,2,4,5-tetracarbonsäure dianhydrid her.

Besonders bevorzugt stellt man beim erfindungsgemäßen Verfahren
(i) Ameisensäure (III);
(ii) als Carbonsäure mit mindestens zwei Kohlenstoffatomen (II) und/oder deren Derivaten Essigsäure, Essigsäuremethylester und/oder Essigsäureanhydrid; und
(iii) als Carbonsäureanhydrid (VII) Propionsäureanhydrid, Buttersäureanhydrid, Acrylsäureanhydrid, Methacrylsäureanhydrid und/oder Benzol-1,2,4,5-tetracarbonsäure dianhydrid
her.

Beim erfindungsgemäßen Verfahren setzt man bei der Umesterung in Schritt (a) den Ameisensäureester (I) und die Carbonsäure (II) im Allgemeinen in einem Verhältnis von 1:1 ein, wobei die relativen Konzentrationen im Reaktionsgemisch gegebenenfalls davon abweichen können. Die Carbonsäure (II) wird dabei als Edukt, als Rückführungsstrom aus Schritt (c) oder als Mischform von beiden zugeführt. Pro Mol an umgesetztem Ameisensäureester (I) sowie an umgesetzter Carbonsäure (II) ensteht entsprechend der Reaktionsgleichung 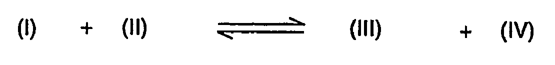 ein Mol Ameisensäure (III) als abzuführendes Produkt sowie ein Mol Carbonsäureester (IV). Da mindestens ein Teil des in Schritt (a) gebildeten Carbonsäureesters (IV) zum entsprechenden Carbonsäureanhydrid (V) carbonyliert wird, wird entsprechend der Reaktionsgleichung 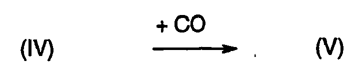 aus einem Mol Carbonsäureester (IV) ein Mol Carbonsäureanhydrid (V) gebildet. Da mindestens ein Teil des in Schritt (b) gebildeten Carbonsäureanhydrids (V) in der Umanhydridisierung unter Zufuhr der Carbonsäure (VI) eingesetzt wird, wird entsprechend der Reaktionsgleichung

(V) + 2 (VI) → (VII) + (II) + (IIa)

aus einem Mol Carbonsäureanhydrid (V) und zwei Mol Carbonsäure (VI) ein Mol Carbonsäureanhydrid (VII), ein Mol Carbonsäure (II) und ein Mol Carbonsäure (IIa), wobei für den Fall des Einsatzes eines symmetrischen Carbonsäureanhydrids (V) entsprechend zwei Mol Carbonsäure (II) gebildet werden, die Carbonsäuren (II) und (IIa) dann identisch sind. Die gebildete Carbonsäure (II) kann als Produkt abgeführt oder der Umesterung in Schritt (a) wieder zugeführt werden.

Tabelle 1 enthält eine Übersicht der bevorzugten Verfahrensvarianten unter Berücksichtigung der stöchiometrischen Verhältnisse, wobei die gebildete Ameisensäure (III) als Bezugsgröße verwendet wurde. Die letzte Spalte enthält die erforderlichen Verfahrensblöcke, wobei der Übersichtlichkeit halber auf die Nennung der optionalen Blöcke zur Ausschleusung möglicher Zwischenprodukte verzichtet wurde.

### Ausführungsform 1: Herstellung von Ameisensäure, Carbonsäureanhydrid (VII) und Essigsäure

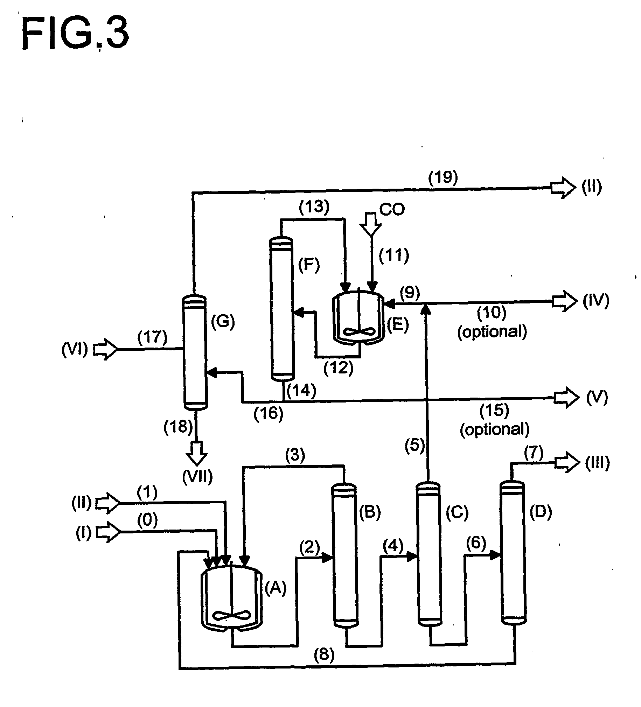

Ein vereinfachtes Verfahrensfließbild ist in Abbildung 3 dargestellt. Ameisensäuremethylester (I) und Essigsäure (II) werden dem Reaktor (A), welcher exemplarisch als Rührkessel dargestellt ist, über Leitung (0) und (1) kontinuierlich zugeführt. Als Reaktor (A) können hierfür jedoch auch andere geeignete Reaktionsapparate, wie beispielsweise weiter oben unter Schritt (a) beschrieben, eingesetzt werden. Im Reaktor (A) findet in Gegenwart des eingesetzten Katalysators die Umesterung zur Ameisensäure (III) und dem Essigsäuremethylester (IV) statt. Das Reaktionsgemisch, welches Ameisensäuremethylester (I), Essigsäure (II), Ameisensäure (III), Essigsäuremethylester (IV) sowie den eingesetzten Katalysator enthält, wird kontinuierlich dem Reaktor (A) entnommen und über Leitung (2) der destillativen Aufarbeitung, welche in Form der Ko-Ionnen (B), (C) und (D) exemplarisch dargestellt ist, zugeführt. Über Leitung (3) werden nicht-umgesetzter Ameisensäuremethylester (I) und eventuell gebildete Leichtsieder dem Reaktor (A) rückgeführt. Ameisensäure (III) wird über Leitung (7) entnommen. Über Leitung (8) werden nicht-umgesetzte Essigsäure (II), Katalysator und eventuell gebildete Hochsieder dem Reaktor (A) rückgeführt. Es versteht sich von selbst, dass je nach Bedarf ein Teil des Stroms (8) zur Vermeidung einer Aufpegelung von Hochsiedern kontinuierlich oder diskontinuierlich ausgeschleust und gegebenenfalls weiter aufgearbeitet werden kann. Essigsäuremethylester (IV) wird über Leitung (5) weitergeleitet. Im Allgemeinen ist es von Vorteil, für die beiden Kolonnen (B) und (C) eine Trennwandkolonne einzusetzen. Strom (3) wird dabei als Kopfstrom, Strom (5) als Seitenstrom und Strom (6) als Sumpfstrom abgeführt.

Über die optionale Leitung (10) ist gegebenenfalls eine Ausschleusung von Essigsäuremethylester (IV) möglich.

Essigsäuremethylester (IV) wird über Leitung (9) dem Reaktor (E), welcher exemplarisch als Rührkessel dargestellt ist, zur Carbonylierung kontinuierlich zugeführt. Als Reaktor (E) können hierfür jedoch auch andere geeignete Reaktionsapparate, wie beispielsweise weiter oben unter Schritt (b) beschrieben, eingesetzt werden. Im Reaktor (E) findet in Gegenwart des eingesetzten Katalysators die Carbonylierung unter Zufuhr von Kohlenmonoxid über Leitung (11) zum Essigsäureanhydrid (V) statt. Das Reaktionsgemisch, welches nicht-umgesetzten Essigsäuremethylester (IV), Essigsäureanhydrid (V) sowie den eingesetzten Katalysator enthält, wird kontinuierlich dem Reaktor (E) entnommen, im Allgemeinen vom Katalysator befreit, beispielsweise in einem Flashverdampfer (der Übersichtlichkeit halber nicht dargestellt), und über Leitung (12) der destillativen Aufarbeitung, welche in Form der Kolonne (F) exemplarisch dargestellt ist, zugeführt. Über Leitung (13) werden nicht-umgesetzter Essigsäuremethylester (IV) und eventuell gebildete Leichtsieder dem Reaktor (E) rückgeführt. Das Sumpfprodukt der Kolonne (F), welches Essigsäureanhydrid (V) sowie eventuell gebildete Hochsieder enthält, wird über Leitung (14) entnommen und im Allgemeinen in einer weiteren Kolonne (der Übersichtlichkeit halber nicht dargestellt) in Essigsäureanhydrid (V) und Hochsieder getrennt. Der Katalysator enthaltende Strom wird in der Regel wieder dem Reaktor (E) rückgeführt. Es versteht sich von selbst, dass je nach Bedarf ein Teil des Hochsieder enthaltenden Stroms zur Vermeidung einer Aufpegelung von Hochsiedern kontinuierlich oder diskontinuierlich ausgeschleust und gegebenenfalls weiter aufgearbeitet werden kann.

Über die optionale Leitung (15) ist gegebenenfalls eine Ausschleusung von Essigsäureanhydrid (V) möglich.

Essigsäureanhydrid (V) wird über Leitung (16) dem Reaktor (G), welcher exemplarisch als Kolonne dargestellt ist, zur Umanhydridisierung kontinuierlich zugeführt. In der Kolonne (G) findet in Gegenwart des eingesetzten Katalysators die Umanhydridisierung unter Zufuhr der Carbonsäure (VI) über Leitung (17) zum Carbonsäureanhydrid (VII) und Essigsäure (II) statt. Das Kopfprodukt der Kolonne (G), welches Essigsäure (II), nicht-umgesetztes Essigsäureanhydrid (V) und eventuell gebildete Leichtsieder enthält, wird über Leitung (19) entnommen und im Allgemeinen in einer weiteren Kolonne (der Übersichtlichkeit halber nicht dargestellt) weiter aufgetrennt. Essigsäure (II) wird als Produkt abgeführt, Essigsäureanhydrid (V) im Allgemeinen wieder zur Kolonne (G) rückgeführt und die Leichtsieder ausgeschleust. Alternativ ist es natürlich auch möglich, das im Kopfprodukt der Kolonne (G) eventuell enthaltene Essigsäureanhydrid (V) mit Wasser zur Essigsäure zur hydrolysieren. Das Sumpfprodukt der Kolonne (G), welches Carbonsäureanhydrid (VII), sowie eventuell den Katalysator und gebildete Hochsieder enthält, wird über Leitung (18) entnommen und im Allgemeinen in einer weiteren Kolonne (der Übersichtlichkeit halber nicht dargestellt) in Carbonsäureanhydrid (V), Katalysator und Hochsieder getrennt. Der Katalysator enthaltende Strom wird in der Regel wieder zur Kolonne (G) zurückgeführt und das Carbonsäureanhydrid (V) als Produkt abgeführt.

Alternativ kann anstelle der Kolonne (G) zur Umanhydridisierung auch eine Hintereinanderschaltung eines Reaktors, wie beispielsweise eines Rührkessels, und eine beziehungsweise mehrere hintereinandergeschaltete Destillationskolonnen zur Aufarbeitung des Reaktionsgemisches eingesetzt werden.

### Ausführungsform 2: Herstellung von Ameisensäure, Carbonsäureanhydrid (VII) und Essigsäure (mit Essigsäurekreislauf)

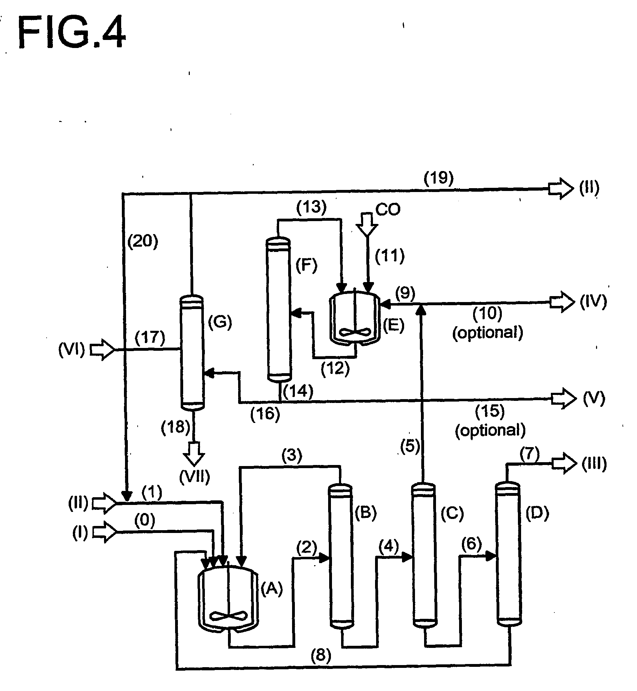

Ein vereinfachtes Verfahrensfließbild ist in Abbildung 4 dargestellt. Die dem Reaktor (A) über Leitung (20) zugeführte Essigsäure (II) stammt zum überwiedenden Teil, bevorzugt vollständig, aus dem Essigsäurekreislauf. Bei Bedarf ist jedoch auch eine Zugabe von zusätzlicher Essigsäure über Leitung (1) möglich. Die Umesterung, die Carbonylierung und die Umanhydridisierung werden wie in Ausführungsform 1 beschrieben durchgeführt, worauf explizit verwiesen sei.

Anstatt die bei der Umanhydridisierung gebildete Essigsäure (II) vollständig über Leitung (19) als Produkt abzuführen wird in dieser bevorzugten Ausführungsform die für die Umesterung in Schritt (a) erforderliche Essigsäure (II) über Leitung (20) wieder dem Reaktor (A) zugeführt, womit sich der Kreislauf schließt. Überschüssige Essigsäure (II) kann natürlich über Leitung (19) als Produkt entnommen werden.

### Ausführungsform 3: Herstellung von Ameisensäure, Carbonsäureanhydrid (VII) und Essigsäureanhydrid (mit Essigsäurekreislauf)

Die ebenfalls bevorzugte Ausführungsform 3 entspricht im Wesentlichen der Ausführungsform 2, jedoch mit dem Unterschied, dass über Leitung (15) ein Teil des gebildeten Essigsäureanhydrids (V) als Produkt abgeführt und nur der zur Aufrechterhaltung des Essigsäurekreislaufes erforderliche Anteil zur Umanhydridisierung weitergeleitet wird. Daher wird in dieser Ausfürhungsform die gesamte Essigsäure, welche bei der Umanhydridisierung gebildet wird, über Leitung (20) zur Umesterung zurückgeführt.

Das erfindungsgemäße Verfahren ermöglicht die Herstellung von (i) Ameisensäure, (ii) einer Carbonsäure mit mindestens zwei Kohlenstoffatomen und/oder deren Derivaten, wie beispielsweise einem Carbonsäureester oder einem Carbonsäureanhydrid, und (iii) eines weiteren Carbonsäureanhydrids, auf Basis einer gut zugänglichen und wirtschaftlich attraktiven Rohstoffbasis. So basieren beispielsweise die besonders bevorzugten Produkte Ameisensäure, Essigsäuremethylester, Essigsäureanhydrid und Essigsäure vollständig auf Synthesegas und somit auf Erdgas als Rohstoff.

Weiterhin ermöglicht das erfindungsgemäße Verfahren eine einfache und kostengünstige Gestaltung der Anlage (niedrige Investitionskosten), einen niedrigen Energieverbrauch und niedrige Betriebskosten. Durch die Kopplung der Herstellung von Ameisensäure und einer Carbonsäure mit mindestens zwei Kohlenstoffatomen und/oder deren Derivaten weist eine nach dem erfindungsgemäßen Verfahren arbeitende Anlage einen deutlich niedrigeren Kapitaleinsatz auf als zwei getrennte Anlagen nach dem Stand der Technik. Insbesondere entfällt bei der Herstellung von Essigsäureanhydrid nach erfindungsgemäßen Verfahren der Umweg über giftiges und in der Herstellung energieintensives Keten.

Das erfindungsgemäße Verfahren vermeidet die Bildung unerwünschter Nebenprodukte infolge Koppelproduktion.

Des Weiteren ermöglicht das erfindungsgemäße Verfahren bei Bedarf auch die Herstellung wasserfreier Ameisensäure und wasserfreier Carbonsäuren, welche eine deutlich geringere Korrosionsaggresivität aufweisen als die wasserhaltigen Verbindungen, somit eine höhere Sicherheit bieten und den Einsatz kostengünstigerer Konstruktionsmaterialen ermöglichen. Durch den gegenüber dem Stand der Technik einfachen und wirtschaftlich attraktiven Zugang zu nahezu wasserfreier Ameisensäure wird eine besonders hohe Ameisensäure-Qualität erreicht. Aufgrund des sehr geringen Restwassergehalts resultiert damit auch ein Vorteil beim Transport und der Lagerung der so hergestellten Ameisensäure.

Ferner bietet das erfindungsgemäße Verfahren ein hohes Maß an Flexibilität auf der Seite der Carbonsäure mit mindestens zwei Kohlenstoffatomen und/oder deren Derivaten, da die relativen Mengen der ausgeschleusten Verbindungen je nach Bedarf in einem bereiten Bereich variieren können. Durch eine zusätzliche Zufuhr eines Alkohols zur Carbonylierungsstufe kann das Verhältnis der Carbonylierungsprodukte zur Ameisensäure erhöht werden. Somit besteht auch in Bezug auf eine Mehrproduktion an Carbonylierungsprodukten und dessen Folgeprodukten ein hohes Maß an Flexibilität.

Bei der bevorzugten Darstellung von Essigsäure und deren Derivaten bietet das erfindungsgemäße den weiteren Vorteil, die Carbonylierung des Essigsäuremethylesters in Abwesenheit von Wasser durchzuführen und somit gegenüber der technisch üblichen Carbonylierung von Methanol durch Vermeidung der Wassergas-Shift-Reaktion eine höhere Ausbeute des eingesetzten Kohlenmonoxids zu erreichen.

Durch den Einsatz des besonders vorteilhaft hergestellten Essigsäureanhydrids als Anhydridisierungsreagenz für Carbonsäuren, wie insbesondere für Propionsäure, Buttersäure, Acrylsäure und Methacrylsäure, und der Rückführung der gebildeten Essigsäure zum Essigsäurekreislauf, ist auch die Herstellung der verschiedensten Carbonsäureanhydride aus den zugrunde liegenden Carbonsäuren besonders vorteilhaft.

**Tabelle 1: Bevorzugte Ausführungsformen unter Berücksichtigung der idealisierten stöchiometrischen Verhältnisse.**

| | Edukte | Produkte | Verfahrensblöcke |
|---|---|---|---|
| 1 | (I): Ameisensäuremethylester | (III): Ameisensäure | A, C, E |
| | | (II): 2 Essigsäure | |
| | (II): Essigsäure | (VII): Carbonsäureanhydrid | |
| | Kohlenmonoxid | | |
| | (VI): 2 Carbonsäure | | |
| 2 | (I): Ameisensäuremethylester | (III): Ameisensäure | A, C, E, F |
| | | (II): Essigsäure | Essigsäure (II) im Kreislauf |
| | Kohlenmonoxid | (VII): Carbonsäureanhydrid | |
| | (VI): 2 Carbonsäure | | |
| 3 | (I): Ameisensäuremethylester | (III): Ameisensäure | A, C, D, E |
| | | (II): ½ Essigsäureanhydrid | Essigsäure (II) im Kreislauf |
| | Kohlenmonoxid | (VII): ½ Carbonsäureanhydrid | |
| | (VI): Carbonsäure | | |

## Patentansprüche

1. Verfahren zur gemeinsamen Herstellung von
(i) Ameisensäure (III);
(ii) einer Carbonsäure mit mindestens zwei Kohlenstoffatomen (II) und/oder deren Derivaten; und
(iii) eines Carbonsäureanhydrids (VII),
**dadurch gekennzeichnet, dass** man
(a) einen Ameisensäureester (I) mit einer Carbonsäure mit mindestens zwei Kohlenstoffatomen (II) zu Ameisensäure (III) und dem entsprechenden Carbonsäureester (IV) umestert;
(b) mindestens einen Teil des in Schritt (a) gebildeten Carbonsäureesters (IV) zum entsprechenden Carbonsäureanhydrid (V) carbonyliert; und
(c) mindestens einen Teil des in Schritt (b) gebildeten Carbonsäureanhydrids (V) mit einer Carbonsäure (VI) unter Bildung eines Carbonsäureanhydrids (VII) und der Carbonsäure (II) umanhydridisiert.

2. Verfahren nach Anspruch 1, **dadurch gekennzeichnet, dass** man
(d) mindestens einen Teil der in Schritt (c) gebildeten Carbonsäure (II) zu Schritt (a) zurückführt.

3. Verfahren nach den Ansprüchen 1 bis 2, **dadurch gekennzeichnet, dass** man die Umanhydridisierung in Schritt (c) in Gegenwart eines sauren oder basischen Ionentauschers oder eines sauren oder basischen Oxids durchführt.

4. Verfahren nach den Ansprüchen 1 bis 2, **dadurch gekennzeichnet, dass** man die Umanhydridisierung in Schritt (c) in Gegenwart einer organischen oder anorganischen Säure, welche einen niedrigeren pKa-Wert als die Carbonsäure (VI) und die Carbonsäure (II) aufweist, durchführt.

5. Verfahren nach den Ansprüchen 1 bis 2, **dadurch gekennzeichnet, dass** man die Umanhydridisierung in Schritt (c) in Gegenwart eines Metallions aus der 1. bis 13. Gruppe des Periodensystems durchführt.

6. Verfahren nach den Ansprüchen 1 bis 5, **dadurch gekennzeichnet, dass** man die Umanhydridisierung in Schritt (c) in einer kontinuierlich betriebenen Destillationskolonne durchführt und die gebildeten Reaktionsprodukte Carbonsäure (II) und Carbonsäureanhydrid (VII) kontinuierlich abführt.

7. Verfahren nach den Ansprüchen 1 bis 6, **dadurch gekennzeichnet, dass** man als Ameisensäureester (I) Ameisensäuremethylester einsetzt.

8. Verfahren nach den Ansprüchen 1 bis 7, **dadurch gekennzeichnet, dass** man als Carbonsäure (II) Essigsäure einsetzt.

9. Verfahren nach den Ansprüchen 1 bis 8, **dadurch gekennzeichnet, dass** man als Carbonsäureanhydrid (VII) Propionsäureanhydrid, Buttersäureanhydrid, Acrylsäureanhydrid, Methacrylsäureanhydrid und/oder Benzol-1,2,4,5-tetracarbonsäure dianhydrid herstellt.

10. Verfahren nach den Ansprüchen 1 bis 9, **dadurch gekennzeichnet, dass** man
(i) Ameisensäure (III);
(ii) als Carbonsäure mit mindestens zwei Kohlenstoffatomen (II) und/oder deren Derivaten Essigsäure, Essigsäuremethylester und/oder Essigsäureanhydrid; und
(iii) als Carbonsäureanhydrid (VII) Propionsäureanhydrid, Buttersäureanhydrid, Acrylsäureanhydrid, Methacrylsäureanhydrid und/oder Benzol-1,2,4,5-tetracarbonsäure dianhydrid
herstellt.

## Claims

1. A process for the joint preparation of
(i) formic acid (III);
(ii) a carboxylic acid having at least two carbon atoms (II) and/or derivatives thereof; and
(iii) a carboxylic anhydride (VII),
which comprises
(a) transesterifying a formic ester (I) with a carboxylic acid having at least two carbon atoms (II) to form formic acid (III) and the corresponding carboxylic ester (IV);
(b) carbonylating at least part of the carboxylic ester (IV) formed in step (a) to form the corresponding carboxylic anhydride (V); and
(c) transanhydriding at least part of the carboxylic anhydride (V) formed in step (b) with a carboxylic acid (VI) to form a carboxylic anhydride (VII) and the carboxylic acid (II).

2. The process according to claim 1, wherein
(d) at least part of the carboxylic acid (II) formed in step (c) is recirculated to step (a) .

3. The process according to claim 1 or 2, wherein the transanhydridation in step (c) is carried out in the presence of an acidic or basic ion exchanger or an acidic or basic oxide.

4. The process according to claim 1 or 2, wherein the transanhydridation in step (c) is carried out in the presence of an organic or inorganic acid which has a pKₐ which is lower than that of the carboxylic acid (VI) and the carboxylic acid (II).

5. The process according to claim 1 or 2, wherein the transanhydridation in step (c) is carried out in the presence of a metal ion from groups 1 to 13 of the Periodic Table.

6. The process according to any of claims 1 to 5, wherein the transanhydridation in step (c) is carried out in a continuously operated distillation column and the reaction products carboxylic acid (II) and carboxylic anhydride (VII) formed are continuously taken off.

7. The process according to any of claims 1 to 6, wherein the formic ester (I) used is methyl formate.

8. The process according to any of claims 1 to 7, wherein the carboxylic acid (II) used is acetic acid.

9. The process according to any of claims 1 to 8, wherein the carboxylic anhydride (VII) prepared is propionic anhydride, butyric anhydride, acrylic anhydride, methacrylic anhydride and/or benzene-1,2,4,5-tetracarboxylic dianhydride.

10. The process according to any of claims 1 to 9, wherein
(i) formic acid (III) is prepared;
(ii) the carboxylic acid having at least two carbon atoms (II) and/or derivatives thereof prepared is/are acetic acid, methyl acetate and/or acetic anhydride; and
(iii) the carboxylic anhydride (VII) prepared is propionic anhydride, butyric anhydride, acrylic anhydride, methacrylic anhydride and/or benzene-1,2,4,5-tetracarboxylic dianhydride.

## Revendications

1. Procédé pour la préparation commune de
(i) acide formique (III) ;
(ii) un acide carboxylique comprenant au moins deux atomes de carbone (II) et/ou ses dérivés ; et
(iii) un anhydride d'acide carboxylique (VII),
**caractérisé**
(a) **en ce qu'**on transestérifie un ester d'acide formique (I) avec un acide carboxylique comprenant au moins deux atomes de carbone (II) en acide formique (III) et l'ester de l'acide carboxylique correspondant (IV) ;
(b) **en ce qu'**on carbonyle au moins une partie de l'ester d'acide carboxylique (IV) formé dans l'étape (a) en anhydride d'acide carboxylique correspondant (V) ; et
(c) **en ce qu'**on trans-anhydride au moins une partie de l'anhydride d'acide carboxylique (V) formé dans l'étape (b) avec un acide carboxylique (VI) avec formation d'un anhydride d'acide carboxylique (VII) et de l'acide carboxylique (II).

2. Procédé selon la revendication 1, **caractérisé**
(d) **en ce qu'**on recycle au moins une partie de l'acide carboxylique (II) formé dans l'étape (c) dans l'étape (a).

3. Procédé selon les revendications 1 à 2, **caractérisé en ce qu'**on réalise la trans-anhydridisation dans l'étape (c) en présence d'un échangeur d'ions acide ou basique ou d'un oxyde acide ou basique.

4. Procédé selon les revendications 1 à 2, **caractérisé en ce qu'**on réalise la trans-anhydridisation dans l'étape (c) en présence d'un acide organique ou inorganique, qui présente un pKa plus bas que celui de l'acide carboxylique (VI) et de l'acide carboxylique (II).

5. Procédé selon les revendications 1 à 2, **caractérisé en ce qu'**on réalise la trans-anhydridisation dans l'étape (c) en présence d'un ion métallique du 1er au 13ème groupe du système périodique.

6. Procédé selon les revendications 1 à 5, **caractérisé en ce qu'**on réalise la trans-anhydridisation dans l'étape (c) dans une colonne de distillation opérée en continu et on évacue en continu l'acide carboxylique (II) et l'anhydride de l'acide carboxylique (VII) en tant que produits de réaction formés.

7. Procédé selon les revendications 1 à 6, **caractérisé en ce qu'**on utilise comme ester d'acide formique (I) de l'ester méthylique de l'acide formique.

8. Procédé selon les revendications 1 à 7, **caractérisé en ce qu'**on utilise de l'acide acétique comme acide carboxylique (II).

9. Procédé selon les revendications 1 à 8, **caractérisé en ce qu'**on prépare comme anhydride d'acide carboxylique (VII) l'anhydride de l'acide propionique, l'anhydride de l'acide butyrique, l'anhydride de l'acide acrylique, l'anhydride de l'acide méthacrylique et/ou le dianhydride de l'acide benzène-1,2,4,5-tétracarboxylique.

10. Procédé selon les revendications 1 à 9, **caractérisé en ce qu'**on prépare
(i) de l'acide formique (III) ;
(ii) comme acide carboxylique comprenant au moins deux atomes de carbone (II) et/ou ses dérivés l'acide acétique, l'ester méthylique de l'acide acétique et/ou l'anhydride de l'acide acétique ; et
(iii) comme anhydride d'acide carboxylique (VII) l'anhydride de l'acide propionique, l'anhydride de l'acide butyrique, l'anhydride de l'acide acrylique, l'anhydride de l'acide méthacrylique et/ou le dianhydride de l'acide benzène-1,2,4,5-tétracarboxylique.
